# EUROPEAN PATENT APPLICATION

(11) **EP 3 595 329 A1**
(43) Date of publication of application: **15.01.2020**
(21) Application number: 19185051.0
(22) Date of filing: 08.07.2019
(51) Int. Cl.: H04R 1/10, G06F 16/635, H04R 25/00, A61B 5/12

(54) **METHOD FOR CUSTOMIZING A HEARING DEVICE AT POINT OF SALE**

(30) Priority: 11.07.2018 US 201816032193
(71) Applicant: Harman International Industries, Inc., Stamford, CT 06901 (US)
(72) Inventor: Butts, Donald Joseph, Westport, CT Connecticut 06880 (US)
(74) Representative: Rummler, Felix

(57) **Abstract**

A system and method for customizing a hearing device to the purchased at the point of sale. The system has a user interface and a test hearing device having a plurality of biometric sensors arranged in a sensor array that contact a user for detecting data representative of an auditory brainstem response. The system creates a customized hearing algorithm using the collected data. The hearing device to be purchased is communicatively coupled to the user interface and the customized hearing algorithm is downloaded to the hearing device to be purchased.

## Description

### TECHNICAL FIELD

The present disclosure is directed to a method for customizing a hearing device and more particularly, a method for customizing a hearing device at its point of sale.

### BACKGROUND

Wireless technology, such as Bluetooth, for exchanging data over short distances requires pairing devices such as smart phones, laptop computers, tablets, wearables and hearing devices such as headphones or earbuds. Traditionally, pairing devices using wireless technology, such as Bluetooth, has been known to be a source of frustration for some users. Frustration arises from problems such as failing to pair, audio hiccups, and dropped connections. Recently, hearing devices such as headphones and earbuds have adopted Bluetooth audio streaming technology that allows a user to customize a configuration of the hearing device for the user's particular needs and desires. However, the process for pairing such devices is subject to some sources of frustration, and may deter a user from seeking out and using customized hearing devices. Customization of products, such as headphones or earbuds, is designed with the purpose of providing a user with an enhanced listening experience. Therefore, it is important to eliminate any source of frustration that may arise during the customization process.

There is a need for a method for customizing a hearing device that prevents a user from being subject to traditional sources of frustration when pairing the devices for customization.

### SUMMARY

A system and method for customizing a hearing device at a point of sale. The system has a user interface, and a test hearing device. In one or more embodiments, the test hearing device may have a plurality of biometric sensors arranged in an array for detecting data representative of an auditory brainstem response. An algorithm designed by the computing device collects the data and configures audio parameters specifically for the user. A hearing device to be purchased by the user purchased communicatively couples with the computing device and receives the algorithm to customize the hearing device to be purchased.

A stand-alone kiosk system for customizing, at a point of sale, a hearing device to be purchased by a user. The stand-alone kiosk system has a test hearing device having a plurality of sensors collecting data in response to a predetermined audio signal played at the hearing device. The test hearing device is communicatively coupled to a user interface to play a test signal and collect the data at the computing device. A customized hearing algorithm is designed by the computing device using the collected data. The hearing device to be purchased is communicatively coupled to the user interface and the customized hearing algorithm is downloaded to the hearing device to be purchased.

### DESCRIPTION OF DRAWINGS

FIG. 1 is a flow diagram of a method for customizing a hearing device at a point of sale;
FIG. 2 is a block diagram of a hearing device with biometric sensors; and
FIG. 3 illustrates a system for the customization of a hearing device at a point of sale.

Elements and steps in the figures are illustrated for simplicity and clarity and have not necessarily been rendered according to any particular sequence. For example, steps that may be performed concurrently or in different order are illustrated in the figures to help to improve understanding of embodiments of the present disclosure.

### DETAILED DESCRIPTION

While various aspects of the present disclosure are described with reference to a particular illustrative embodiment, the present disclosure is not limited to such embodiments, and additional modifications, applications, and embodiments may be implemented without departing from the present disclosure. In the figures, like reference numbers will be used to illustrate the same components. Those skilled in the art will recognize that the various components set forth herein may be altered without varying from the scope of the present disclosure.

Any one or more of the servers, receivers, or devices described herein include computer executable instructions that may be compiled or interpreted from computer programs created using a variety of programming languages and/or technologies. In general, a processing unit (such as a microprocessor) receives instructions, for example from a memory, a computer-readable medium, or the like, and executes the instructions. The processing unit includes a non-transitory computer-readable storage medium capable of executing instructions of a software program. The computer readable medium may be, but is not limited to, an electronic storage device, a magnetic storage device, an optical storage device, an electromagnetic storage device, a semi-conductor storage device, or any suitable combination thereof. Any one or more the devices herein may rely on firmware, which may require updates from time to time to ensure compatibility with operating systems, improvements and additional functionality, security updates or the like. Connecting and networking servers, receivers or devices may include, but are not limited to, Bluetooth, SATA, Wi-Fi, lightning, Ethernet, UFS, 5G, etc., and one or more servers, receivers, or devices may operate using a dedicated operating system, multiple software programs and/or platforms for interfaces such as graphics, audio, wireless networking, enabling applications, integrating hardware of vehicle components, systems, and external devices such as smart phones, tablets, and other systems to name just a few.

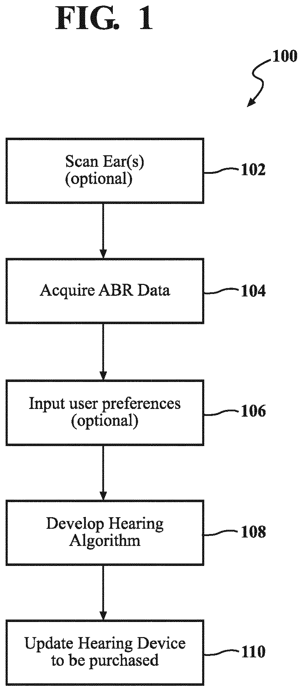

FIG. 1 is a diagram of a method 100 for customizing a hearing device at a point of sale. The hearing device may be a set of headphones that are worn on the outside of a user's ears, a set of earbuds that are worn in-ear or rest on an outer ear without sealing an auditory surface. The hearing device may be wired or wireless. When a user enters a retailer, or similar location, looking to purchase a hearing device such as a new set of earbuds or earphones that are inserted into or around a user's ears. The method 100 is carried out by a user by way of a user interface having a user input, a processor having storage capabilities and the capability to communicatively connect (wirelessly or by wire) to the hearing device to be purchased, a test hearing device and an optional scanning device. The method 100 involves optionally scanning 102 the user's ear(s) to capture the geometry of inner and outer canals in the ear to find an optimal size and fit for the new set of earbuds. Scanning 102 maybe accomplished using a scanner having 3D scanning capability and provides a non-invasive means to determine fit for the user. Such a non-invasive scanner may be easily placed in retail locations. This step is considered optional because a standard set of earbuds or earphones, whose size is not a customizable feature does not require a scan of the user's ears.

Data representative of the user's hearing loss threshold, preference for a particular signal processing algorithm, and other data is acquired 104 from the user. This is accomplished by having the user wear a headset or hearing device that includes biometric sensors that collect data while the user listens to an audio signal, such as music, while wearing the hearing device. In particular, auditory brainstem response data is acquired 104 using the headset having biometric sensors which will be discussed later herein and which is described in detail in US patent 9,787,274, incorporated in its entirety by reference herein. The hearing device used to collect the data may be a set of headphones or earbuds that is to be purchased by the user having biometric sensors therein capable of collecting the data. Alternatively, it may be a separate hearing device, such as a test hearing device, dedicated to the retail space and used only for the purpose of acquiring auditory brainstem response data from the user. Any settings that are determined from the data acquired by the test hearing device will later be transferred to the hearing device that is to be purchased by the user.

The user's hearing loss threshold may also be measured using standard hearing threshold tests that result in an audiogram. Again, any settings that are determined from the data acquired by the test hearing device will later be transferred to the hearing device that is to be purchased by the user.

Additional to the electrical activity being measured by the biometric sensors, the user may also provide input preferences 106, such as preferences for different signal processing algorithms, when prompted. The preferences input by the user may further be used to define a hearing algorithm that is developed 108 for customization of the hearing device to be purchased. Some examples of preferences input by the user may include, but are not limited to, correction of impedance differences using an audio chirp excitation, designing customized trainings in response to data collected that might indicate a learning disability due to hearing loss, and customized head-related transfer functions (HRTF). A head-related transfer function is a response that characterizes how an ear receives a sound from a point in space. There may also be static measurements captured from accelerometers on the test hearing device such as heart rate, heart rate variability, surface electromyography (EMG) to measure mouth movement, electrooculography (EOG) to measure eye movement. Health training programs may be developed and customized based on the measurements taken by the test hearing device.

The customized hearing algorithm is developed 108 for the hearing device to be purchased based on the user's ABR data and, optionally, the user's input preferences. In the situation where the hearing device is also the device to be purchased, the developed algorithm may be immediately available at the hearing device. In the situation where the test hearing device is used to collect the data, the hearing device to be purchased will be paired to the user interface and the developed hearing algorithm will be transferred to the hearing device from the teste hearing device.

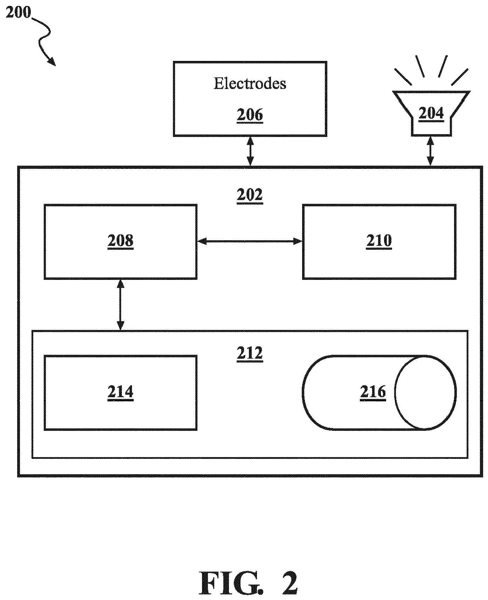

FIG. 2 is a block diagram of an example test hearing device 200, or hearing device to be purchased by a user, that may be used for determining an algorithm for customizing audio parameters associated with hearing characteristics of a user. The test hearing device 200 has a computing device 202, one or more speakers 204, and one or more electrodes 206. The computing device 202 includes a processing unit 208, input/output devices 210 and a memory device 212. The memory device 212 has a calibration application 214 configured to interact with a database 216 and creates an algorithm that is developed to configure audio parameters specifically for a user and provide the configured audio parameters to a hearing device purchased by the user. The electrodes 206 in the test hearing device may include one or more electroencephalography (EEG) electrodes implemented in a sensor array.

The hearing device 200 may be an earphone having a body that may be shaped to fit the user's ear canal as customized from the scanning described above as in cases where the test headset is also the set of headphones or earbuds that is to be purchased by the user. Alternatively, the earphone may be included in an over-the-ear headphone, around-the-ear headphone, also to be purchased by the user. Still further, in instances where the hearing device to be purchased does not incorporate the biometric sensors used in collecting data representative of user's hearing loss threshold, the test headset may be an over-the-ear or around-the-ear headphone that is used strictly for testing, and the results of the method are then transferred to the hearing device to be purchased.

Electrodes 206 are integrated into the body of the test headset. The user, while wearing the headset having biometric sensors, listens to a test audio signal, such as a predetermined music signal or the like. The electrodes in the headset measure electrical activity produced in a brain of the user in response to the audio signal. As discussed above, the electrodes may incorporate biometric sensors, such as EEG and are configured to contact the user's scalp, ear canal, or other portions of the user's head or body. Advantageously, the measured electrical activity does not rely on the user's direct input in response to the stimuli. It is measured directly by the biometric sensors.

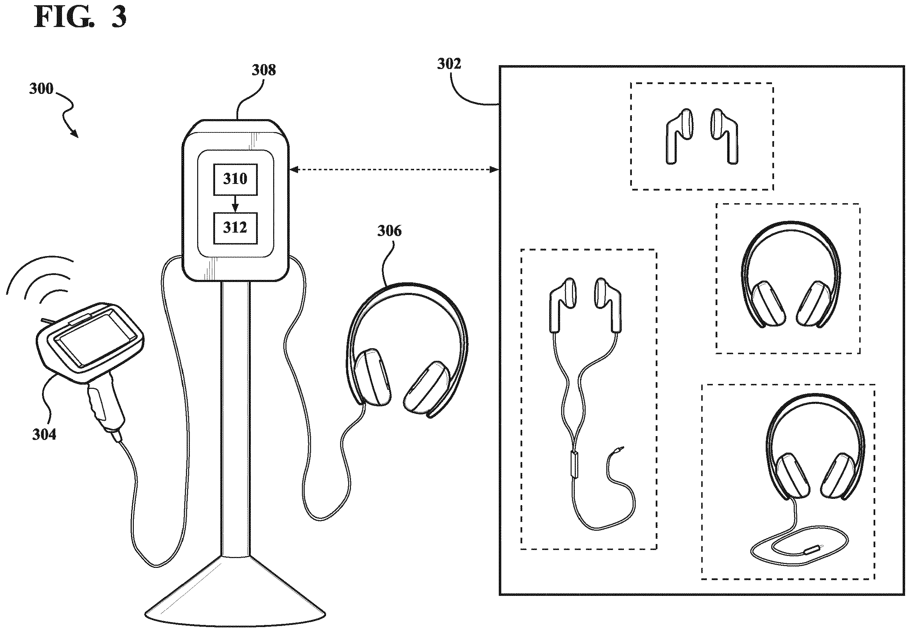

Referring now to FIG. 3, a system 300 is shown for customizing a hearing device 302 to be purchased by a user. Typically, the system 300 is located in a retail space, such as a brick and mortar location, a kiosk, or a pop-up location, and may be monitored by a sales associate. The system 300 is accessible to the user and may or may not be monitored by a sales associate or retail representative. The sales associate or retail representative may help the user through the process on site at the retail location. Alternatively, the user may be able to use the system 300, such as a standalone kiosk, to complete the customization process on their own.

The hearing device 302 to be purchased by a user may be a set of headphones, wired or wireless, a set of earbuds, wired or wireless, etc. Some examples of which are shown in FIG. 3. A scanning device 304 may be used to perform an optional scan on an ear or ears of the user to determine size and shape of the user's ear(s) for customized fit of the hearing device. A test hearing device 306, which may or may not be the same as the hearing device 302 to be purchased by a user, is worn by a user, plays an audio signal, and measures (using electrodes in contact with the user) hearing loss thresholds by way of auditory brainstem response. The hearing threshold test does not require input or manual responses from the user. When the hearing device to be purchased 302 includes the electrodes necessary to collect the auditory brainstem response, it may also be used as the test hearing device 306. When the hearing device 302 to be purchased does not have electrodes, a separate test hearing device 306 must be used to collect the auditory brainstem response.

A stand-alone system 308 provides a user interface for initiating and facilitating the customization process. Each of the hearing device 302 to be purchased, the scanning device 304 and the test hearing device 306 are in communication (wired or wireless) with the stand-alone unit 308. The stand-alone unit has a user interface 310, such as a touchscreen, tablet, keyboard, or the like, and processing/memory capability 312, that enables the user or the retail sales associate to interact with the stand-alone unit 308.

Interacting with the stand-alone unit 308, the user or retail associate helping the user, is able to initiate the customization, collect data from the optional scan and audio test signal, analyze the collected data. Further, the user may provide preferences, such as different signal processing algorithms that may be selected by the user, for customizing the hearing device 302 to be purchased. Once all the data has been collected and analyzed, the hearing device 302 to be purchased is paired with the processor 312 of the stand-alone device 3018 and the customized algorithms are downloaded to the hearing device 302 to be purchased.

In the foregoing specification, the present disclosure has been described with reference to specific exemplary embodiments. Various modifications and changes may be made, however, without departing from the scope of the present disclosure as set forth in the claims. The specification and figures are illustrative, rather than restrictive, and modifications are intended to be included within the scope of the present disclosure. Accordingly, the scope of the present disclosure should be determined by the claims and their legal equivalents rather than by merely the examples described.

For example, the steps recited in any method or process claims may be executed in any order and are not limited to the specific order presented in the claims. The equations may be implemented with a filter to minimize effects of signal noises. Additionally, the components and/or elements recited in any apparatus claims may be assembled or otherwise operationally configured in a variety of permutations and are accordingly not limited to the specific configuration recited in the claims.

Benefits, other advantages and solutions to problems have been described above with regard to particular embodiments; however, any benefit, advantage, solution to problem or any element that may cause any particular benefit, advantage or solution to occur or to become more pronounced are not to be construed as critical, required or essential features or components of any or all the claims.

The terms "comprise", "comprises", "comprising", "having", "including", "includes" or any variation thereof, are intended to reference a non-exclusive inclusion, such that a process, method, article, composition or apparatus that comprises a list of elements does not include only those elements recited, but may also include other elements not expressly listed or inherent to such process, method, article, composition or apparatus. Other combinations and/or modifications of the above-described structures, arrangements, applications, proportions, elements, materials or components used in the practice of the present disclosure, in addition to those not specifically recited, may be varied or otherwise particularly adapted to specific environments, manufacturing specifications, design parameters or other operating requirements without departing from the general principles of the same.

## Claims

1. A system for customizing a hearing device at a point of sale, the system comprising:
a user interface having a computing device;
a test hearing device having a plurality of sensors collecting data in response to a predetermined audio signal played at the hearing device and collected by the computing device;
an algorithm designed by the computing device using the collected data that configures audio parameters specifically for the user; and
a hearing device to be purchased by the user, the hearing device to be purchased communicatively couples with the computing device and receives the algorithm to customize the hearing device to be purchased.

2. The system as claimed in claim 1 wherein the plurality of sensors further comprises a plurality of biometric sensors arranged in a sensor array that contact a user for detecting data representative of an auditory brainstem response.

3. The system as claimed in claim 1 wherein the user interface further comprises prompts that require user input preferences collected as data to be used by the algorithm to further configure audio parameters specifically for the user.

4. The system as claimed in claim 2 wherein the biometric sensors are electroencephalography electrodes.

5. The system as claimed in claim 2 wherein the hearing device to be purchased has a plurality of biometric sensors and may be used as the test hearing device.

6. The system as claimed in claim 1 further comprising a scanner that scans a geometry of an ear of the user and communicates the scan data to the computer device wherein a size of the hearing device to be purchased is determined specifically for the user.

7. The system as claimed in 1 wherein the hearing device to be purchased communicatively couples with the computing device through a wireless connection initiated by the user, or wherein the hearing device to be purchased communicatively couples with the computing device through a wireless connection initiated by a sales associate.

8. A method for customizing a hearing device, the method comprising the steps of:
collecting, at a user interface in a retail space, data representative of a hearing loss threshold;
developing, in a non-transitory computer readable medium comprising a program executed by a processor incorporated in the user interface, a hearing algorithm in response to the collected data;
communicatively coupling a hearing device to be purchased with the user interface;
downloading the hearing algorithm to the hearing device to be purchased.

9. The method as claimed in claim 8 wherein the step of collecting, at a user interface in a retail space, data representative of a hearing loss threshold further comprises the steps of:
playing a predefined audio signal through a test hearing device worn by a user, the test hearing device having a plurality of biometric sensors arranged in an array and in contact with the user;
sensing an auditory brainstem response of the user from the plurality of biometric sensors as the predefined audio signal is played, the sensed auditory brainstem response is collected as data representative of a hearing loss threshold;
and preferably prompting the user to input preferences to be used by the algorithm to further configure audio parameters specifically for the user.

10. The method as claimed in claim 8 further comprising the steps of:
capturing a geometry of inner and outer ear canals of an ear of a user;
communicating the captured geometry to the user interface; and
determining, at the user interface, a size for the hearing device to be purchased,
wherein the step of capturing a geometry preferably is performed on the user by a sales associate in the retail space.

11. The method as claimed in claim 8 wherein the step of communicatively coupling a hearing device to be purchased with the user interface is initiated by a sales associate in the retail space.

12. A stand-alone kiosk system for customizing, at a point of sale, a hearing device to be purchased by a user; the system comprising:
a user interface having a computing device, a user input mechanism, and a display screen;
a test hearing device having a plurality of sensors collecting data in response to a predetermined audio signal played at the hearing device, the test hearing device is communicatively coupled to the user interface to play a test signal and collect the data at the computing device;
a customized hearing algorithm designed by the computing device using the collected data, the hearing device to be purchased is communicatively coupled to the user interface and the customized hearing algorithm is downloaded to the hearing device to be purchased.

13. The system as claimed in claim 12 wherein the plurality of sensors is a plurality of biometric sensors arranged in a sensor array that contact a user for detecting data representative of an auditory brainstem response, wherein the hearing device to be purchased preferably further comprises biometric sensors arranged in a sensor array and stands in lieu of the test hearing device.

14. The system as claimed in claim 12 further comprising a scanner communicatively coupled to the user interface and capable of capturing a geometry of an inner and an outer ear canal of the user for recommending, at the user interface, a size of the hearing device to be purchased by the user, wherein the scanner preferably is a non-invasive, three dimensional scanner.

15. The system as claimed in claim 12 further comprising user preferences, input by the user at the user interface, to be used with the data representative of an auditory brainstem response in the customized hearing algorithm.
